# EUROPEAN PATENT SPECIFICATION

(11) **EP 3 353 284 B1**
(45) Date of publication and mention of the grant of the patent: **04.11.2020**
(21) Application number: 16847651.3
(22) Date of filing: 23.09.2016
(51) Int. Cl.: C12N 1/14, C12P 7/16, A01N 63/30, A01P 1/00, A01P 3/00, A01P 7/04

(54) **BIOACTIVE FUNGI**
BIOAKTIVE PILZE
CHAMPIGNONS BIOACTIFS

(30) Priority: 24.09.2015 AU 2015903908
(43) Date of publication of application: 01.08.2018
(73) Proprietor: Agriculture Victoria Services Pty Ltd, Bundoora, VIC 3083 (AU)
(72) Inventor: MANN, Ross, Wendouree, Victoria 3355 (AU); SPANGENBERG, German Carlos, Bundoora, Victoria 3083 (AU); AUER, Desmond, South Morang, Victoria 3058 (AU); KRILL, Christian, Reservoir, Victoria 3073 (AU); SAWBRIDGE, Timothy Ivor, Coburg, Victoria 3058 (AU); EDWARDS, Jacqueline, Brunswick, Victoria 3056 (AU); ROCHFORT, Simone Jane, Reservoir, Victoria 3073 (AU)
(74) Representative: Grund, Martin
(86) International application number: PCT/AU2016/050888
(87) International publication number: WO 2017/049353

(56) References cited:
- WO-A1-2016/125153
- WO-A2-2014/165174
- US-A1- 2013 137 131
- Kamila Tomoko Yuyama ET AL: "Daldinia eschscholtzii (Ascomycota, Xylariaceae) isolated from the Brazilian Amazon: taxonomic features and mycelial growth conditions", Acta Amazonica, 1 March 2013 (2013-03-01), pages 1-8, XP055574729, DOI: 10.1590/S0044-59672013000100001 Retrieved from the Internet: URL:http://www.scielo.br/pdf/aa/v43n1/v43n 1a01.pdf
- STADLER, M. ET AL.: 'A polyphasic taxonomy of Daldinia (Xylariaceae' STUDIES IN MYCOLOGY vol. 77, 2014, pages 1 - 143, XP055373061
- ANKE, H. ET AL.: 'Secondary metabolites with nematicidal and antimicrobial activity from nematophagous fungi and Ascomycetes' CANADIAN JOURNAL OF BOTANY (FIFTH INTERNATIONAL MYCOLOGICAL CONGRESS, SECT. E-H vol. 73, no. SUPPL., 1995, pages S932 - S939, XP002914554

## Description

### Field of the Invention

The present invention relates to fungi, in particular fungal endophytes of *Daldinia* spp., compounds produced by the fungi, uses of the fungi, uses of compounds produced by the fungi and similar compounds, and related methods.

### Background of the Invention

Microbes represent an invaluable source of genes and compounds that have the potential to be utilised in a range of industrial sectors. Scientific literature gives numerous accounts of microbes being the primary source of antibiotics, immunosuppressants, anticancer agents, cholesterol-lowering drugs and agricultural chemicals, in addition to their use in environmental decontamination and in the production of food and cosmetics. A relatively unexplored group of microbes known as endophytes, which reside in the tissues of living plants, offer a particularly diverse source of novel compounds and genes that may provide important benefits to society, and in particular, agriculture.

Endophytes often form mutualistic relationships with their hosts, with the endophyte conferring increased fitness to the host, often through the production of defence compounds. At the same time, the host plant offers the benefits of a protected environment and nutriment to the endophyte. Bioprotectant endophytes that have been developed and commercialised include *Neotyphodium* species that produce insecticidal alkaloids, including peramine (a pyrrolopyrazine) and the lolines (pyrrolizidines). These compounds can accumulate to high levels *in planta* where they act as potent feeding deterrents against a range of insect pests. The insecticidal compounds, destruxins, have also been well characterised as secondary metabolites of fungi. Another antimicrobial compound of fungi is the peptaibols, produced by *Trichoderma virens, Quercus suber, Trichoderma citrinoviridae,* that show antifungal activity against a range of plant pathogens, including *Biscogniauxia mediterranea* and *Apiognomonia quercine.*

Recent discoveries highlight the diversity of applications of endophytes, such as in the energy (e.g. biofuels) sector, and the agricultural sector where fungal species have been identified that produce volatile biocidal metabolites which show application as fumigants. For instance, the fungus *Muscodor albus* from *Cinnamomum zeylanicum* in Honduras produces a suite of volatile antimicrobial compounds that are effective against soil borne pathogens, and this has enabled development of a commercial preparation which has been evaluated as a biological alternative (e.g. mycofumigant) to soil fumigation. Furthermore, the discovery of the endophytic fungus *Ascocoryne sarcoides,* which produces a variety of hydrocarbons commonly found in diesel, petrol and biodiesel, offers mankind a potential alternative to fossil fuels.

There is an increasing need to identify alternative soil and quarantine fumigants, as a number of chemicals have environmental or resistance issues. For instance, the widely used fumigant methyl bromide has been identified as a significant ozone depleter, contributing to the degradation of the Antarctic ozone hole, which has led to increased UV exposure and higher incidence of skin cancer in the southern hemisphere. Furthermore, the multi-purpose fumigant phosphine has shown increased incidence of resistance in insect populations following fumigation, which has biosecurity implications and market access issues globally (e.g. stored grain).

It is estimated that there are up to one million endophytic organisms which may possess genes and compounds that offer enormous benefits to agriculture, particularly in the area of pest and disease management. As such, there exists a need to isolate and identify these endophytes, and characterise the compounds responsible for their activity.

US2013137131 describes the production of biocidal volatile chemicals by e.g. *Daldinia* fungi.

It is an object of the present invention to overcome, or at least alleviate, one or more of the difficulties or deficiencies associated with the prior art.

### Summary of the Invention

This patent application describes fungi of *Daldinia* sp., and applications of the fungi and compounds produced thereby, and analogues thereof, which exhibit *inter alia* broad spectrum activity against important agricultural pests and pathogens. Antibiotic compounds responsible for the activity are characterised.

In one aspect, the present invention provides a substantially purified or isolated fungus of *Daldinia* spp., consisting of *Daldinia* sp. (U254). A representative sample was deposited at the National Measurement Institute with accession number V15/028236 on 22 September 2015.

In its natural environment, the fungus may be an endophyte, i.e. live mutualistically within a plant. Alternatively, the fungus may be an epiphyte, i.e. grow attached to or upon a plant. The fungus may be a heterotroph that uses organic carbon for growth, more particularly a saprotroph that obtains nutrients by consuming detritus.

The fungus of the present invention may in its natural environment be associated with a plant of the genus *Pittosporum.* In a preferred embodiment, the plant of the genus *Pittosporum* is a plant of the species *Pittosporum bicolor.* For example, the *Daldinia* sp. isolate (U254) as deposited at the National Measurement Institute with accession number V15/028236 on 22 September 2015 was isolated from a plant of the species *Pittosporum bicolor* located in a cool temperate rainforest within the Yarra Ranges of Victoria, Australia. By 'associated with' in this context is meant that the fungus lives on, in or in close proximity to the plant. For example, it may be endophytic, for example living within the internal tissues of the plant, or epiphytic, for example growing externally on the plant.

Described is a method of culturing a fungus of *Daldinia* spp. Said method may include growing said fungus in a culture medium including a source of carbohydrates. In a preferred embodiment, the fungus of *Daldinia* spp. may be as hereinbefore described.

The source of carbohydrates may be a starch/sugar-based agar or broth such as potato dextrose agar (PDA), potato dextrose broth or half strength potato dextrose agar (HPDA) or a cereal-based agar or broth such as oatmeal agar (OA) or oatmeal broth. Other sources of carbohydrates can include endophyte agar (ENDO), Murashige and Skoog with 20% sucrose (MS-SUC), half V8 juice/half PDA (V8PDA), water agar (WA) and yeast malt extract agar (YME).

The fungus may be cultured in a culture medium including potato dextrose or oatmeal, for example PDA, HPDA, OA, potato dextrose broth, oatmeal broth or YME. A preferred culture medium may be selected from one or more of OA, PDA and YME. Most preferably, the fungus may be cultured in a culture medium including oatmeal.

The fungus may be cultured under aerobic or anaerobic conditions, for example microaerophilic conditions.

The fungus may be cultured for a period of approximately 1 to approximately 100 days, more preferably from approximately 1 to approximately 50 days more preferably from approximately 1 to approximately 10 to 20 days.

The fungus may be cultured in dark and/or light conditions. For example, the fungus may be cultured in continual darkness, or under a regime of approximately 12 hours dark and approximately 12 hours light, or under a regime of approximately 12 hours darklight and approximately 12 hours dark. Preferably, the fungus is cultured in continual darkness.

The fungus may also be cultured under conditions of constant temperature, or under conditions of a range of temperatures. Preferably, the fungus is cultured at room temperature.

The fungus may be cultured in a bioreactor. By a 'bioreactor' is meant a device or system that supports a biologically active environment, such as a vessel in which is carried out a chemical process involving fungi of the present invention and/or products thereof. The chemical process may be aerobic or anaerobic. The bioreactor may have a volume ranging in size from milliliters to cubic metres, for example from approximately 50 millilitres to approximately 50,000 litres. The bioreactor may be operated via batch culture, batch feed culture, perfusion culture or continuous culture, for example continuous culture in a stirred-tank bioreactor. Fungi cultured in the bioreactor may be suspended or immobilised.

In a preferred embodiment, the method may include the further step of recovering one or more organic compounds produced by the fungus from fungal cells, from the culture medium or from air space associated with the culture medium or fungus. For example, the organic compound(s) may be recovered from intracellular tissues, from the culture medium into which the fungus may secrete liquids, or from the air space into which the fungus may secrete vapours. Most preferably, the organic compound(s) may be recovered from the air space into which the fungus may secrete vapours.

Vapours may arise directly from the fungus or from the secreted liquids which transition between vapour and liquid phases.

The step of recovering the organic compound(s) is preferably done by separating cells from the culture medium or capturing vapours associated with the culture medium or fungus.

Preferably the organic compound(s) is then isolated or purified by a method selected from the group consisting of gas chromatography, liquid chromatography, fractional distillation, cryogenic distillation, membrane separation and absorption chromatography, such as pressure, vacuum or temperature swing adsorption.

The organic compound(s) may be identified by mass spectrometry. A preferred method includes the use of a triple quadrupole mass selective detector operating in electron impact ionization mode at 70 eV. Acquisitions may be carried out over any appropriate mass range, for example a mass range of m/z 29 to 330 for small compounds, with a scan time of 200 milliseconds. This may include a comparison of mass fragmentation patterns against a reference library, e.g. NIST. The mass spectrometer may be coupled with a gas chromatograph (i.e. GC-MS).

By an 'organic compound' is meant a chemical compound, the molecules of which contain the element carbon.

In a preferred embodiment, the organic compound may be a hydrocarbon such as a volatile hydrocarbon or a liquid hydrocarbon. Most preferably, the organic compound(s) may be a volatile hydrocarbon.

By a 'hydrocarbon' is meant an organic compound containing, *inter alia,* the elements carbon and hydrogen.

The term 'volatile' in this context is meant an organic compound which can evaporate or sublimate at standard laboratory temperature and pressure. Volatile organic compounds include those with a high vapour pressure, low boiling point and/or low molecular weight.

In a preferred embodiment, the organic compound(s) may have a molecular weight of about 16 to about 500 g/mole. Preferably, the organic compound(s) has a molecular weight of about 16 to about 400 g/mole, and more preferably of about 16 to about 330 g/mole.

Accordingly, in a preferred embodiment, the organic compound may be selected from one or more of:
(a) the group consisting of: acetaldehyde, pentane, ethanol, 3-pentanol, acetone, 2,3-butanedione, isobutanol, ethyl acetate, isovaleraldehyde, 5,5-dimethyl-1,3-cyclopentadiene, 5,5-dimethyl-1,3-cyclopentadiene, bicyclo[4.1.0]hept-2-ene, 1-methyl-1,4-cyclohexadiene, (Z)-3-methyl-1,3,5-hexatriene, toluene, 4-methylphenol, 3-methyl-1-butanol, 2-methyl-1-butanol, (1Z)-3-methyl-1,3,5-hexatriene, (2Z)-3-methyl-1,3,5-hexatriene, p-xylene, styrene, dimethylcyclopentadiene, 1,4-cyclohexadiene, 4-heptyn-2-ol, 4-ethyl-1-octyn-3-ol, 2,2,4,6,6-pentamethyl-heptane, phenylethyl alcohol, guaiene, [1S-(1α,4α,7α)]-1,2,3,4,5,6,7,8-octahydro-1,4,9,9-tetramethyl-4,7-methanoazulene, (E)-2-pentene, (Z)-2-pentene, 1-methyl-cyclohexene, 3-methyl-cyclohexene, tricyclene, α-pinene, 1-isopropyl-3-methylcyclohexane, p-menth-3-ene, 1-methyl-4-(1-methylethyl)-cyclohexene, 2-carene, p-menth-1-ene, cymene, terpenes and C7 aliphatic/aromatic unsaturated hydrocarbons; and
(b) a compound characterisable by about a base peak selected from the group consisting of a m/z of: 43.2, 59.1, 67.0, 68.1, 71.1, 79.0, 79.1, 81.0, 82.0, 91.0, 95.0, 97.0, 98.0, 108.0, 109.9, 115.0, 124.0, 132.9 and 192.8, or a base peak which is ± 0.1 of any of the foregoing, when analysed by mass spectrometry.

In a more preferred embodiment, the organic compound may be selected from one or more of:
(a) the group consisting of: acetaldehyde, pentane, ethanol, 3-pentanol, acetone, ethyl acetate, isovaleraldehyde, 1-methyl-1,4-cyclohexadiene, toluene, 4-methylphenol, 3-methyl-1-butanol, 2-methyl-1-butanol, styrene, phenylethyl alcohol, (E)-2-pentene, (Z)-2-pentene, 1-methyl-cyclohexene, 3-methyl-cyclohexene, tricyclene, α-pinene,1-isopropyl-3-methylcyclohexane, p-menth-3-ene, 1-methyl-4-(1-methylethyl)-cyclohexene, 2-carene, p-menth-1-ene, cymene, terpenes and C7 aliphatic/aromatic unsaturated hydrocarbons; and
(b) a compound characterisable by about a base peak selected from the group consisting of a m/z of: 43.2, 59.1, 67.0, 79.0, 79.1, 91.0, 95.0, 97.0 and 108.0, or a base peak which is ± 0.1 of any of the foregoing, when analysed by mass spectrometry.

In an even more preferred embodiment, the organic compound may be selected from one or more of the group consisting of acetaldehyde, 3-pentanol, isovaleraldehyde, 3-methyl-1-butanol, 2-methyl-1-butanol and 1,4-cyclohexadiene.

That is, the at least one organic compound may be one or a combination of any two or more or all of the above. For example, preferred combinations include isovaleraldehyde and 3-pentanol, and either of isovaleraldehyde and 3-pentanol with any one or more of acetaldehyde, 1,4-cyclohexadeine and 2-methyl-1-butanol.

By a 'base peak' in this context is meant the most intense or tallest peak in a mass spectrum obtained from an analysis of a compound by mass spectrometry. A base peak may be characteristic of a compound. Analysis of a compound by mass spectrometry is preferably performed as herein before described. An organic compound characterisable by a base peak may also be characterisable by significant minor peaks in the mass spectrum. Preferred significant minor peaks include those listed in Table 3.

In another aspect, the present invention provides a method of producing one or more organic compounds, the method including culturing a fungus of *Daldinia* spp. in a culture medium under conditions suitable to produce said organic compound. The culturing of the fungus in a culture medium under conditions suitable to produce said organic compound may be as hereinbefore described.

Preferably the organic compound is a compound as hereinbefore described.

The organic compound(s) produced by a fungus of *Daldinia* spp. can be used as a biofuel or biofuel precursor, in biofumigation or bioprotection, or in the cosmetic or pharmaceutical industry (for example as a surfactant).

For example, the organic compound(s) may be used as a fumigant. In particular, the organic compound(s) may be used for quarantine and preshipment (QPS), structural or soil fumigation.

The organic compound(s) may be used to fumigate various commodities, including but not limited to, stored grain, soil, timber, buildings, fresh produce and import/export goods.

Fumigants containing an organic compound(s) produced according to the present invention may be applied by any suitable method. Suitable methods for applying fumigants would be familiar to a person skilled in the art. For example, fumigants containing an organic compound(s) of the present invention may be applied by application directly to the fumigation area and/or commodity to be fumigated. This may include application by spraying, gassing, clouding, wetting, injecting, sublimating and dusting. For example, fumigants containing an organic compound(s) of the present invention may be applied by direct injection into a fumigation area. Application may be with or without a carrier gas such as CO₂ and air, and with or without heating. Application may also be by moisture activation of a pelleted form, with or without a binding agent such as metal binding agents of aluminium, zinc and calcium.

The organic compound(s) may be effective against pests and diseases, including but not limited to insects such as grain borers and beetles, including grain borers and beetles selected from the group consisting of Lesser Grain Borer (*Rhyzopertha dominica*), Sawtooth Grain Beetle (*Oryzaephilus suinamensis*), Rust Red Flour Beetle (*Tribolium castaneum*) and Flat Grain Beetle (*Crryptolestes ferrugineus*). They may have benefits selected from the group consisting of being safer, less damaging to the environment, less susceptible to resistance and faster acting than commonly used fumigants.

For example, insect mortality may be evident after approximately 3 to approximately 10 days of fumigation, more preferably after approximately 3 to approximately 7 days of fumigation. With currently used fumigants, such as phosphine, insect mortality may not be evident until up to approximately 20 days of fumigation.

As hereinbefore described, the one or more organic compounds may be produced by a fungus of *Daldinia* spp; for example by culturing a fungus of *Daldinia* spp. and recovering one or more organic compounds produced by the fungus from fungal cells, from the culture medium or from air space associated with the culture medium or fungus.

Alternatively, the organic compound(s) may be synthesised or otherwise obtained, and compositions thereof where desirable may be manufactured by admixture. For example, one or more organic compounds that are substantially identical to one or more organic compounds produced by a *Daldinia* spp. fungus, or are analogues thereof, may be provided, and may be mixed with other components to form a composition. The one or more organic compounds may be synthesised by suitable chemical reactions. Other components may include, for example, further organic compounds or other materials commonly used in compositions, such as binders, carriers, propellants, azeotropes, surfactants, etc., depending on the desired application. These materials and methods of manufacture would be familiar to a skilled worker in the art.

By 'substantially identical' is meant for example, the same, or a stereoisomer, regioisomer, skeletal isomer, positional isomer, functional group isomer, structural isomer, conformational isomer, tautomer, or other isomer, isotopic variant, derivative or salt thereof. By an 'analogue' is meant a similar compound differing in respect of a certain structural component. The term encompasses both 'substantially identical' compounds and derivatives, along with other similar compounds. By a 'derivative' is meant an organic compound obtained from, or regarded as derived from, another compound. Examples of derivatives include compounds where the degree of saturation of one or more bonds has been changed (e.g., a single bond has been changed to a double or triple bond) or wherein one or more atoms are replaced with a different atom or functional group. Examples of different atoms and functional groups may include, but are not limited to, hydrogen, halogen, oxygen, nitrogen, sulphur, hydroxy, alkoxy, alkyl, alkenyl, alkynyl, amine, amide, ketone and aldehyde.

By way of example, acetoin is a structural isomer of ethyl acetate (i.e. same molecular formula). Similarly, acetoin differs from ethyl acetate by way of a certain structural component (i.e. -OCH₂CH₃ *cf.* -CHOHCH₃) and may thus be considered to be an analogue thereof.

The organic compound produced by culturing a *Daldinia* spp. fungus in a culture medium under conditions suitable to produce said organic compound, of which the organic compound(s) of this aspect of the invention are substantially identical thereto or analogues thereof, may be an organic compound as hereinbefore described, produced as hereinbefore described.

In a preferred embodiment, the at least one organic compound is a volatile organic compound.

In another preferred embodiment, the at least one organic compound has a molecular weight of about 16 to about 500 g/mole, more preferably of about 16 to about 400 g/mole, and even more preferably of about 16 to about 330 g/mole.

Thus, preferably the at least one organic compound may be selected from:
(a) the group consisting of: acetaldehyde, pentane, ethanol, 3-pentanol, acetone, 2,3-butanedione, isobutanol, ethyl acetate, isovaleraldehyde, 5,5-dimethyl-1,3-cyclopentadiene, 5,5-dimethyl-1,3-cyclopentadiene, bicyclo[4.1.0]hept-2-ene, 1-methyl-1,4-cyclohexadiene, (Z)-3-methyl-1,3,5-hexatriene, toluene, 4-methylphenol, 3-methyl-1-butanol, 2-methyl-1-butanol, (1Z)-3-methyl-1,3,5-hexatriene, (2Z)-3-methyl-1,3,5-hexatriene, p-xylene, styrene, dimethylcyclopentadiene, 1,4-cyclohexadiene, 4-heptyn-2-ol, 4-ethyl-1-octyn-3-ol, 2,2,4,6,6-pentamethyl-heptane, benzaldehyde, phenylethyl alcohol, guaiene, [1S-(1α,4α,7α)]-1,2,3,4,5,6,7,8-octahydro-1,4,9,9-tetramethyl-4,7-methanoazulene, (E)-2-pentene, (Z)-2-pentene, 1-methyl-cyclohexene, 3-methyl-cyclohexene, tricyclene, α-pinene,1-isopropyl-3-methylcyclohexane, p-menth-3-ene, 1-methyl-4-(1-methylethyl)-cyclohexene, 2-carene, p-menth-1-ene, cymene, terpenes, C7 aliphatic/aromatic unsaturated hydrocarbons, 4-hydroxy-2-butanone, butyric acid, methylenecyclohexane, 4-methylcyclohexane, 1,3-cycloheptadiene, butyraldehyde, spiro[2.4]hepta-4,6-diene and acetoin; and
(b) a compound characterisable by about a base peak selected from the group consisting of a m/z of: 43.2, 59.1, 67.0, 68.1, 71.1, 79.0, 79.1, 81.0, 82.0, 91.0, 95.0, 97.0, 98.0, 108.0, 109.9, 115.0, 124.0, 132.9 and 192.8, or a base peak which is ± 0.1 of any of the foregoing, when analysed by mass spectrometry.

In a more preferred embodiment, the at least one organic compound may be selected from:
(a) the group consisting of: acetaldehyde, pentane, ethanol, 3-pentanol, acetone, ethyl acetate, isovaleraldehyde, 1-methyl-1,4-cyclohexadiene, toluene, 4-methylphenol, 3-methyl-1-butanol, 2-methyl-1-butanol, styrene, benzaldehyde, phenylethyl alcohol, (E)-2-pentene, (Z)-2-pentene, 1-methyl-cyclohexene, 3-methyl-cyclohexene, tricyclene, α-pinene, 1-isopropyl-3-methylcyclohexane, p-menth-3-ene, 1-methyl-4-(1-methylethyl)-cyclohexene, 2-carene, p-menth-1-ene, cymene, terpenes and C7 aliphatic/aromatic unsaturated hydrocarbons, 4-hydroxy-2-butanone, butyric acid, methylenecyclohexane, 4-methylcyclohexane, 1,3-cycloheptadiene, butyraldehyde, spiro[2.4]hepta-4,6-diene and acetoin; and
(b) a compound characterisable by about a base peak selected from the group consisting of a m/z of: 43.2, 59.1, 67.0, 79.0, 79.1, 91.0, 95.0, 97.0 and 108.0, or a base peak which is ± 0.1 of any of the foregoing, when analysed by mass spectrometry.

In an even more preferred embodiment, the at least one organic compound may be selected from one or more of the group consisting of: acetaldehyde, 3-pentanol, isovaleraldehyde, 3-methyl-1-butanol, 2-methyl-1-butanol, 1,4-cyclohexadiene, 4-hydroxy-2-butanone, butyric acid, methylenecyclohexane, 4-methylcyclohexane, 1,3-cycloheptadiene, butyraldehyde, spiro[2.4]hepta-4,6-diene and acetoin.

In another more preferred embodiment, the at least one organic compound may be selected from one or more of the group consisting of acetaldehyde, 3-pentanol, isovaleraldehyde and acetoin.

That is, the at least one organic compound may be one or a combination of any two or more or all of the above. For example, preferred combinations include isovaleraldehyde and/or acetoin with 3-pentanol, and any one or more of isovaleraldehyde, acetoin and 3-pentanol with any one or more of acetaldehyde, 1,4-cyclohexadeine, 2-methyl-1-butanol, 1,3-cycloheptadeine and 4-methylcyclohexene.

In another aspect, the present invention provides a method for inhibiting an insect or a micro-organism including exposing the insect or micro-organism to an organic compound, a composition for use as a fumigant or a biocidal composition as hereinbefore described.

In a preferred embodiment, the insect is a pest of stored grain, including but not limited to *Tribolium castaneum, Rhyzopertha dominica, Cryptolestes ferrugineus* and *Oryzaephilus suinamensis.*

Also in a preferred embodiment, the micro-organism is a fungus selected from one or more of the genus *Fusarium, Botrytis, Alternaria* or *Rhizoctonia,* such as species *Fusarium verticillioides, Botrytis cinerea, Alternaria alternata* and *Rhizoctonia cerealis,* and a bacteria of the genus *Pseudomonas* such as species *Pseudomonas syringae.*

As used herein, 'an insect' and 'a micro-organism' is taken to include a population.

Inhibition of an insect or micro-organism may be by way of a decrease in a normal activity. For an insect, this may include, for example, prevention or reduction of insect proliferation, growth or breeding. In preferred embodiments, the biocidal composition causes insect mortality, for example by fumigation as hereinbefore described. The amount to which the insect is exposed may be from about 100 to about 200, preferably about 50 to about 200, more preferably about 20 to about 200, microlitres of organic compound per litre of environment (e.g. container, vessel, silo etc.). For a micro-organism, inhibition may include prevention or reduction of proliferation or growth.

For example, a reduction in growth may be evident after approximately 1 to approximately 10 days of exposure, e.g. fumigation, more preferably after approximately 1 to approximately 4 days of exposure.

The present invention will now be more fully described with reference to the accompanying Examples and drawings. It should be understood, however, that the description following is illustrative only and should not be taken in any way as a restriction on the generality of the invention described above.

### Brief Description of the Figures

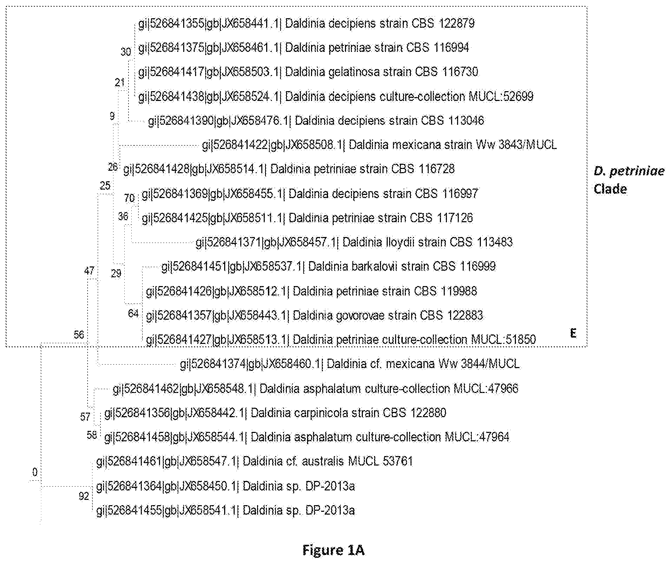

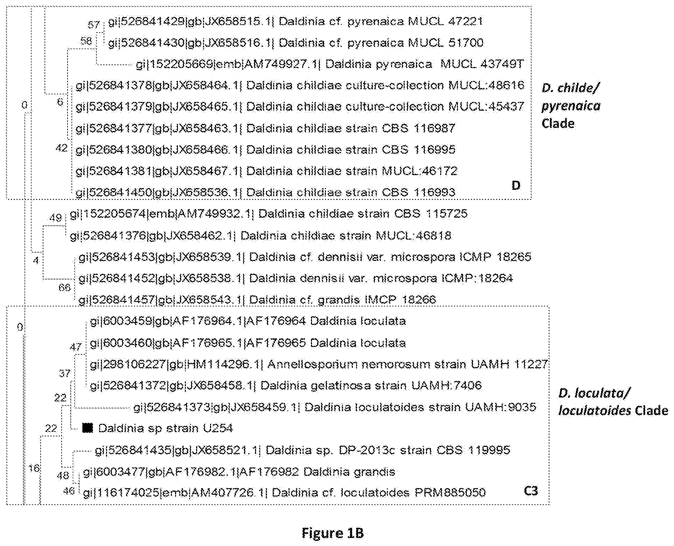

Figures 1A-1F depict a phylogenetic tree showing the phylogenetic relationships among *Daldinia* sp (U254) and *D*. *eschscholtzii, D. concentrica, D. childiae*/*pyrenaica, D. vernicosa*/*loculata*/*novae-zelandiae, D. petriniae,* along with their respective allies. Isolate U254 is represented by a solid black square in Figure 1B.

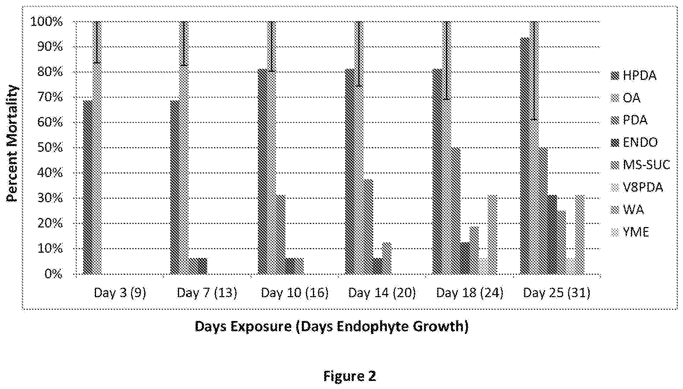

Figure 2 shows the percentage mortality of *Tribolium castaneum* following exposure to *Daldinia* sp. (U254) in insect assays, when grown on different media (bars from left to right: HPDA - half strength potato dextrose agar, OA - oatmeal agar, PDA, ENDO - endophyte agar, MS-SUC - Murashige and Skoog with 20% sucrose, V8PDA - half V8 juice/half PDA, WA - water agar, YME - yeast malt extract agar). Bars represent the least significant difference (LSD) at a significance level of 5%.

### Detailed Description of the Embodiments

### Example 1 - Endophyte identification

A broad-based endophyte discovery program was undertaken across Victoria and the Northern Territory, Australia. Over 1000 endophytic fungi were isolated from over 100 plant species. Preliminary screens for bioactivity identified approximately 250 isolates of varying degrees of bioactivity.

### Example 2 - Isolation of Daldinia sp. (U254)

A single endophytic fungal isolate of *Daldinia* sp. (U254) was collected from a cool temperate rainforest within the Yarra Ranges of Victoria, Australia. The isolate was collected as an endophyte of foliar tissue from *Pittosporum bicolor* in the Yarra Ranges.

The host plant, *Pittosporum* is comprised of around 200 species, and is widely distributed across Australasia, Oceania, East Asia and parts of Africa. A number of species are extensively cultivated globally and grown as ornamental plants. *Pittosporum bicolor* is a shrub or tree of commonly 3 to 10 metres in height when mature. Leaves are alternate, usually narrowly ovate to narrowly oblong, commonly 3 to 8 centimetres long and 5 to 18 millimetres wide. Leaf margins are flat to recurved. The leaf apex is subacute to obtuse. The lower leaf surface is usually densely hairy and rarely glabrescent with age. Petioles are commonly 2 to 3 millimetres in length. Flowers are usually terminal, solitary or a few together. Sepals are commonly 3 to 6 millimetres long, with few to many hairs. Petals are commonly 8 to 11 millimetres long, yellow with purple-maroon markings. Ovaries are hairy. Capsules are globose, usually 5 to 8 millimetres long, hairy, grey, and with valves dark on the inner face. Seeds are few to many, and red in colour.

### Example 3 - Morphological Characterisation of the Daldinia sp. (U254)

Colonies of *Daldinia* sp. (U254) were grown on oatmeal agar (OA) and reached the edge of a 9 centimetre Petri dish in 3-5 days. They were at first whitish, felty, azonate, with diffuse margins, becoming grey with dark grey, green and white tones; reverse cream to yellow. Co-indiogenous structures of normal *Daldinia* types were not observed, instead the vegetative mycelium differentiated into a network of stromatic structures, composed of black, thick-walled, incrusted hyphae, producing thick-walled chlamydospore-like structures, remaining sterile. The hyphae were branched, hyaline and smooth.

### Example 4 - Molecular Characterisation of the Daldinia sp. (U254)

A phylogenetic analysis of *Daldinia* sp. (U254) was undertaken by sequence homology comparison of the 5.8S-ITS ribosomal gene region. The isolate was grown on OA, from which mycelia were harvested and used for DNA extraction using the Qiagen Blood and Tissue Kit according to manufacturer's instructions (QIAGEN, Germany). The ribosomal gene region was amplified using a KAPA2G Robust PCR kit with the universal primers ITS5 (5'-GGAAGTAAAAGTCGTAACAAGG-3') and ITS4 (5'-TCCTCCGCTTATTGATATGC-3'). The PCR conditions were as follows: 94 °C, 5 minutes (1 cycle); 94 °C, 30 seconds; 50 °C, 30 seconds; 72 °C, 2 minutes (35 cycles); 72 °C, 7 minutes (1 cycle). The amplicon was then submitted to Macrogen (Seoul, South Korea) for purification and sequencing.

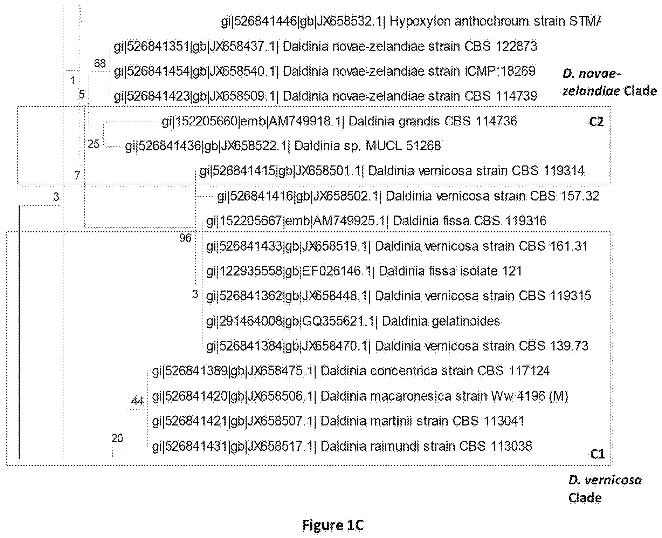

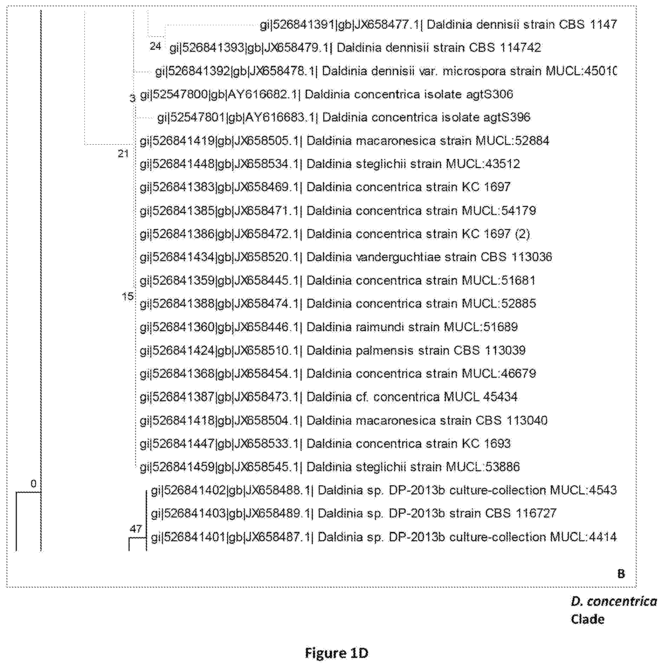

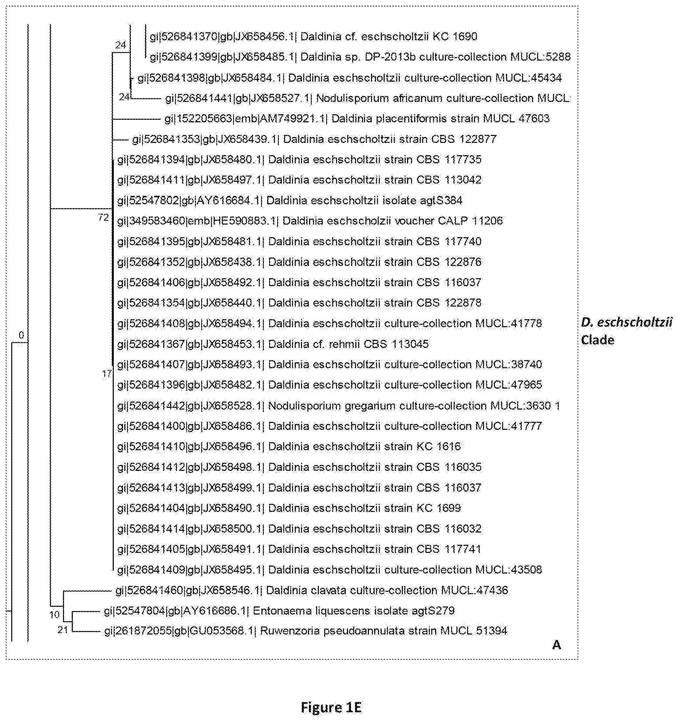

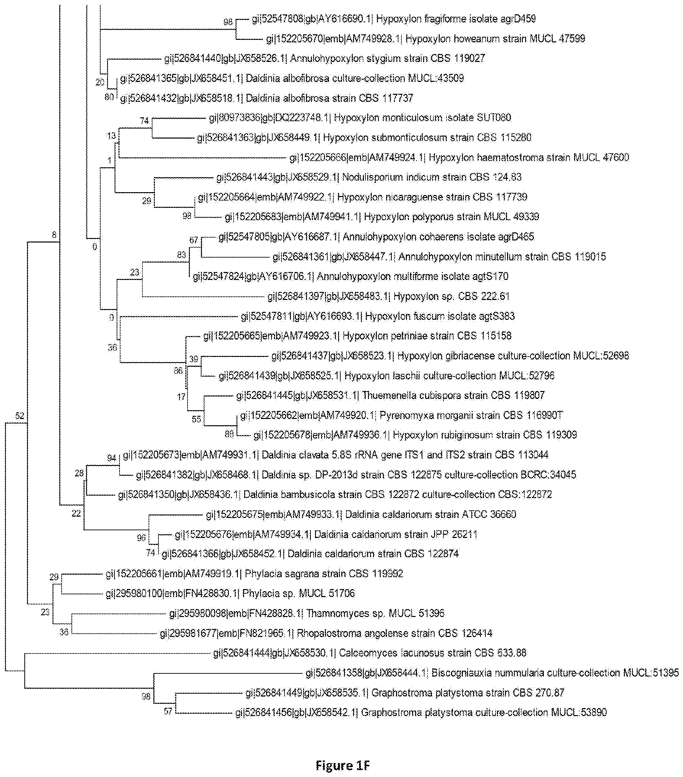

Sequence homology searching against the NCBI nucleotide database (Blastn - type specimen parameter) identified closely related fungi to isolate U254, of which *Daldinia* species were the closest match (e.g. 96%) (Table 1). These sequences, and sequences of other *Daldinia* species (type specimens) were collected and aligned in MEGA5 using the Muscle algorithm (sourced from Stadler *et al.*)*.* Based on the sequence alignment, phylogenetic relationships were inferred using Maximum Likelihood (ML) and Maximum Parsimony (MP) analyses. For ML analysis, phenograms were obtained using the nearest-neighbour-interchange method, applying the Tamura-Nei model. For MP analysis, phenograms were obtained using the Subtree-Pruning-Regrafting algorithm (search level 3). In both analyses, alignment gaps and missing data were eliminated from the dataset (Complete deletion option) and the confidence of branching was assessed by computing 1000 bootstrap replications. The phylogenetic trees had similar topology to Stadler *et al.* (2014), forming 5 distinct *Daldinia* clades that comprised *Daldinia eschscholtzii* (A), *Daldinia concentrica* (B), *Daldinia vernicosa* (C1), *Daldinia novae-zelandiae* (C2), *Daldinia loculata*/*loculatoides* (C3), *Daldinia childe*/*pyrenaica* (D), and *Daldinia petrinae* (E). The *Daldinia* sp. (U254) clustered in the *Daldinia loculatoides* and *Daldinia loculata* clade (clade C3), along with *Daldinia grandis* and *Daldinia gelatinosa* (red square - *Daldinia* sp. U254) (Figure 1). Despite the low bootstrap support (consistent with Stadler *et al.,* 2014) isolate U254 is clearly a *Daldinia* species, and likely belongs to the *Daldinia loculatoides*/*loculata* clade. As it does not produce anamorphic structures *in vitro* like other species in the clade, it is a new *Daldinia* species.

**Table 1 - Comparison of ribosomal gene sequences of Daldinia sp. (U254) with other closely related species (type specimens) from NCBI sequence homology search.**

| **NCBI Accession** | **Genus** | **Species** | **Max Score** | **E Value** | **Query Coverage** | **Identity** |
|---|---|---|---|---|---|---|
| gb\|JX658510.1\| | *Daldinia* | *palmensis* | 835 | 0 | 87% | 96% |
| gb\|JX658517.1\| | *Daldinia* | *raimundi* | 830 | 0 | 87% | 96% |
| gb\|JX658479.1\| | *Daldinia* | *dennisii* | 830 | 0 | 87% | 96% |
| gb\|JX658441.1\| | *Daldinia* | *decipiens* | 752 | 0 | 88% | 93% |
| gb\|JX658537.1\| | *Daldinia* | *barkalovii* | 623 | 5e-176 | 72% | 93% |
| gb\|KC968938.1\| | *Hypoxylon* | *fraxinophilum* | 623 | 5e-176 | 100% | 86% |
| gb\|KC968922.1\| | *Hypoxylon* | *liviae* | 593 | 4e-167 | 99% | 85% |
| gb\|KC968930.1\| | *Hypoxylon* | *gibriacense* | 584 | 3e-164 | 98% | 85% |
| gb\|KC968934.1\| | *Hypoxylon* | *laminosum* | 573 | 6e-161 | 98% | 85% |

### Example 5 - Bioactivity of Daldinia sp. (U254)

*In vitro* bioassays were established to test the bioactivity of *Daldinia* sp. (U254) against a range of insect pests (*Tribolium castaneum*) and plant pathogenic fungi (*Botrytis cinerea, Alternaria alternata* and *Rhizoctonia cerealis*)*.* The bioassays used a 9 centimetre split Petri plate, which contained an impermeable septum through the centre of the plate, which completely separated the plate into two halves. This only permitted volatile compounds to pass over the septum to act against the test organism, and prevented any direct contact between the endophyte (or its liquid exudates) and the test organism.

For the insect assay *Daldinia* sp. (U254) was inoculated on to Petri plates containing OA, half potato dextrose agar (HPDA), potato dextrose agar (PDA), endophyte agar (ENDO), Murashige and Skoog with 20% sucrose (MS-SUC), half V8 juice/half PDA (V8PDA), water agar (WA) and yeast malt extract agar (YME). An agar plug containing actively growing mycelia from the endophyte was placed approximately 13 millimetres from the edge of the plate (i.e. on one half of the plate). The endophytic fungus was allowed to grow at room temperature (in the dark) for 6 days. Subsequently, the insect pests were inoculated on to the other half of the plate by placing four insects onto filter paper and a food source (wheat meal). Plates were sealed with low-density polyethylene (LDPE) plastic film (approximately 0.01 millimetres thick) and covered in aluminium foil (i.e. kept in the dark). After 3, 7, 10, 14, 18 and 25 days the viability was assessed by measuring the activity of the insect (non-active - dead; active - alive).

Measurements were compared to the control and expressed as percentage mortality versus the control (Figure 2). Data were analysed using ANOVA as performed in GenStat, version 14. The experiment was fully randomised with 4 replicates. *Daldinia* sp. (U254) caused mortality rates of 100% for *Tribolium castaneum* following 3 days exposure to the endophyte when grown on OA. The insecticidal bioactivity of *Daldinia* sp. (U254) was significantly greater on OA than all other media at 3 and 7 days exposure, with HPDA providing equivalent activity at 10 to 25 days exposure (maximum insecticidal activity - 94%).

For the plant pathogenic fungus assay, *Daldinia* sp. (U254) was inoculated on to split Petri plates containing PDA. An agar plug containing actively growing mycelia from the endophyte was placed approximately 13 millimetres from the edge of the plate (i.e. on one half of the plate). The endophytic fungus was allowed to grow at room temperature (in the dark) for 6 days. Subsequently, the plant pathogenic fungi were inoculated on to the other half of the plate by placing an agar plug containing actively growing hyphae approximately 13 millimetres from the edge of the plate. Plates were sealed with LDPE plastic film (approximately 0.01 millimetres thick) and covered in aluminium foil. After 2, 5, 7, and 11 days the viability was assessed by measuring the radial growth of the plant pathogenic fungus.

Measurements were compared to the control and expressed as percentage inhibition versus the control (Table 2). Data were analysed using ANOVA as performed in GenStat, version 14. The experiment was fully randomised with 3 replicates. *Daldinia* sp. (U254) completely inhibited the growth of *Botyrtis cinerea* and *Rhizoctonia cerealis* at all time points, while the growth of *Alternaria alternata* was inhibited by a minimum of 76.3 %.

**Table 2 - Percentage inhibition of Daldinia sp. (U254) in plant pathogenic fungus assays against 3 plant pathogenic fungi, Botrytis cinerea, Alternaria alternata and Rhizoctonia cerealis.**

| **Pathogen** | **Day 2** | **Day 5** | **Day 7** | **Day 11** |
|---|---|---|---|---|
| *Botrytis cinerea* | 100.0% | 100.0% | 100.0% | 100.0% |
| *Alternaria alternata* | 100.0% | 85.7% | 76.3% | 81.8% |
| *Rhizoctonia cerealis* | 100.0% | 100.0% | 100.0% | 100.0% |

### Example 6 - Volatolome of Daldinia sp. (U254)

Gases were analysed in the head space above cultures of *Daldinia* sp. (U254). The isolate was cultured under microaerophilic conditions, which consisted of growing the fungus on OA and YME slopes in 20 ml glass vials, with an agar:air ratio of 1:2.5. Vials were sealed with a screw cap lid with polytetrafluoroethylene (PTFE) septum, and grown for 9 days at room temperature.

A head space solid phase microextraction (SPME) was performed to capture volatiles produced by *Daldinia* sp. (U254). A StableFlex fibre (Supelco) coated with 75 micrometre Carboxen/PDMS was used to absorb volatiles from the head space of vials. Automated sampling was performed by an Gerstel Multi Purpose Sampler using the proprietary Maestro software. The fibre was conditioned at 270 °C for 60 minutes prior to commencement of activities and for 30 minutes between each sample. For each sample the fibre was inserted into the vial and incubated at room temperature for 7 minutes to absorb volatiles, after which the fibre was inserted into a splitless injection port of an Agilent 7890 gas chromatography (GC) system where the contents were thermally desorbed (250 °C for 6 minutes) onto an Agilent DB-624 capillary column (25 metres x 250 micrometres id., 1.4 micrometre film thickness, column 1) coupled to an Agilent inert fused silica 2 metres x 250 micrometres id (no film) (column 2) via a purged union controlled by an Agilent Auxiliary Electronic Pressure Control module. The column oven was programmed as follows: 35 °C (3 minutes), 3 °C/minute to 200 °C, then 25 °C/minute to 250 °C (2 minutes). The carrier gas was helium with a constant flow rate of 1 millilitre / minute for column 1 and 1.8 millilitre / minute for column 2. The GC was interfaced with an Agilent 7000 GC/MS triple quadrupole mass selective detector (mass spectrometer, MS) operating in electron impact ionization mode at 70 eV. The temperature of the transfer line was held at 280 °C during the chromatographic run. The source temperature was 280 °C. Acquisitions were carried out over a mass range of mz 29 to 330, with a scan time of 200 milliseconds.

Initial identification of the volatiles produced by *Daldinia* sp. (U254) was made through library comparison using standard chemical databases. Secondary confirmatory identification was made by comparing mass spectral data of authentic standards with data of the fungal volatiles. All chemical names in this report follow the nomenclature of the standard chemical databases. In all cases, uninoculated control vials were also analysed and the compounds found therein were subtracted from those appearing in the vials supporting fungal growth. Tentative identification of the fungal volatiles was based on observed mass spectral data as compared to those in these chemical databases and those of authentic standards (where possible).

The GC-MS analysis (0 to 65 minutes) identified 31 volatile metabolites produced by *Daldinia* sp. (U254) when grown for 9 days on OA and YME at room temperature (Table 3). The metabolites produced by *Daldinia* sp. (U254) were representative of a number of structural classes, including alcohols (e.g. ethanol, 3-methyl-1-butanol), aldehydes (e.g. acetaldehyde, isovaleraldehyde), esters (e.g. ethyl acetate), terpenes and C7 aliphatic/aromatic unsaturated hydrocarbons and their associated derivatives (C7, e.g. 1-methylcyclohexene, 1-methyl-1,4-cyclohexadiene, toluene). The C7 compounds represent the major structural class within the volatolome, composing approximately one third of the compounds produced.

**Table 3 - GC-MS headspace analysis of the volatile compounds produced by Daldinia sp. (U254) when grown on OA and YME for 9 days at room temperature.**

| **Compound** | **Match** | **RT** | **BP (m/z)** | **Significant Minor Ions (% of BP)** | **Relative Intensity** |
|---|---|---|---|---|---|
| Acetaldehyde | 3 | 3.51 | 44.1 | 43 (44.3), 42 (13.89) | + |
| Pentane | 2 | 4.95 | 43.1 | 42.1 (63.35), 41.1 (44.56), 57.1 (26.32) | + |
| Ethanol | 3 | 5.39 | 45.1 | 46.1 (24.49), 43.1 (14.42) | +++ |
| mz67,68,53@6.14min | | 6.14 | 67.0 | 68.1 (85.3), 53.1 (40.02) | + |
| Acetone | 2 | 6.21 | 43.1 | 58.1 (66.27) | + |
| mz59,42,60,41@9.60min | | 9.62 | 59.1 | 42.1 (43.59), 60.1 (30.15), 41.2 (19.08) | + |
| Ethyl acetate | 3 | 11.03 | 43.1 | 61.1 (39.57), 70.1 (33.77) | + |
| mz43,41,42@13.48min | | 13.50 | 43.2 | 41.2 (70.95), 42.1 (64.33) | + |
| Isovaleraldehyde | 3 | 13.96 | 44.0 | 43.0 (93.80), 41.2 (89.88), 58.1 (81.88) | + |
| mz79,77,94@15.33min | | 15.33 | 79.0 | 94 (59), 77 (56.52), 91.1 (38.45) | + |
| mz79,77,94@16.67min | | 16.66 | 79.0 | 77 (52.78), 94.1 (44.48), 91 (21.11) | + |
| mz79,77,94@17.04min | | 17.04 | 79.0 | 77.1 (48.88), 94 (43.42) | ++ |
| mz79,77,94@17.97min | | 17.97 | 79.0 | 94 (58.92), 77 (58.26), 91 (35.98) | + |
| mz79,77,94@18.53min | | 18.53 | 79.0 | 77.1 (58.47), 94 (57.44), 91.1 (35.01) | +++ |
| mz79,77,94@19.02min | | 19.02 | 79.1 | 77 (58.65), 94 (56.92), 91 (34.56) | +++ |
| Toluene | 3 | 19.42 | 91.0 | 92 (51.12), 65 (10.14) | +++ |
| mz79,91,77@20.01min | | 20.02 | 79.0 | 91 (1306.57), 77 (963.93), 94 (48.68) | ++ |
| 3-Methyl-1-butanol | 3 | 20.29 | 55.0 | 70.1 (80.81), 42.1 (57.06), 43 (47.19) | ++ |
| 2-Methyl-1-butanol | 3 | 20.47 | 56.0 | 57.1 (89), 70.1 (70.36), 41.1 (45.55) | + |
| mz79,77,94@20.87min | | 20.87 | 79.1 | 77 (73.45), 94 (66.91), 91 (30.89) | +++ |
| mz79,77,94@21.55min | | 21.55 | 79.0 | 77 (61.57), 94 (54.52) | +++ |
| 1-Methyl-1,4-cyclohexadiene | 3 | 22.03 | 79.1 | 77 (75.27), 94 (55.41), 91 (28.26) | ++ |
| mz91,92@23.09min | | 23.10 | 91.0 | 92 (55.2), 0 (0) | +++ |
| Phenol, 4-methyl- | 2 | 24.47 | 108.0 | 107 (62.04), 79 (12.57), 77 (9.94) | ++ |
| mz108,79,77@26.18min | | 26.19 | 108.0 | 79 (63.92), 77 (38.91) | + |
| Styrene | 2 | 28.73 | 104.0 | 103 (42.47), 78 (34.55) | + |
| Benzaldehyde | 3 | 34.68 | 105.0 | 106 (95.77), 77 (65.67), 51 (16.49) | + |
| mz95,67,110@36.60min | | 36.61 | 95.0 | 67.1 (41.38), 110 (29.01) | + |
| mz79,108@38.88min | | 38.91 | 79.0 | 108 (87.72), 77.1 (51.5) | + |
| mz97,69,125@42.54min | | 42.54 | 97.0 | 69.2 (32.09), 125 (21.95) | + |
| Phenylethyl alcohol | 3 | 44.11 | 91.0 | 92 (50.08), 121.9 (23.13), 64.9 (12.21) | + |

| | | | | | |
|---|---|---|---|---|---|
| 1 - Fragmentation pattern matches spectra of compound in NIST library (>75%) 2 - Fragmentation pattern matches spectra of compound in NIST library (>90%) 3 - Fragmentation pattern matches spectra and retention time of authentic standard + - significant peak; ++ - major peak; +++ - dominant peak | | | | | |

For large scale trapping, the fungus was inoculated onto 120 standard OA plates and incubated in the dark for 3 days at 25 °C. An 18.5 litre desiccator was baked at 150 °C for 3 days and allowed to cool inside a laminar flow cabinet. The fungal plates were stacked without lids inside the desiccator in an overlapping manner so as to prevent them from touching the agar of the plates, while allowing maximum air exchange. The desiccator was sealed using LDPE plastic film and wrapped in aluminium foil (i.e. in the dark). The tap opening in the lid was closed using a silicone rubber stopper in which two holes were drilled. Two pieces of Teflon tubing were passed through these holes, one leading all the way to approximately 2 centimetres above the desiccator bottom, and a shorter one to approximately 5 centimetres from the desiccator top acting as the air inlet. A Supelco VOST Stack Sampling Tube (Tenax TA:Petroleum Charcoal, 2:1) was attached to the inlet (outside the desiccator) to purify the air entering the system. To the outlet, a 30 × 5 centimetre glass column filled with approximately 100 grams of anhydrous sodium sulphate was attached to remove excess moisture from the gas phase. A second Sampling Tube was attached to the end of the glass column to trap volatiles produced by the fungal cultures (Fungal Volatiles Trap, FVT). Negative pressure was applied to the whole system to result in a flow rate of approximately 40-60 millilitres/minute, channelling the culture headspace through the second trap to adsorb any volatiles present. The system was left undisturbed for 7 days at room temperature, after which the trap was removed and placed inside the oven of an Agilent 7890 GC and connected to the GC APC (auxiliary pressure control) module. High purity Nitrogen was flushed through the trap at an initial rate of approximately 25 millilitres/minute. A piece of stainless steel tubing was connected to the outlet of the trap, leading into a glass vial sitting on top of a metal block partially submerged in liquid nitrogen that acted as a cold trap. The FVT was dry purged at ambient temperature for 30 minutes, then heated to 200 °C to thermally desorb the volatiles, which were recovered in the cold trap. The FVT was then reconnected to the desiccator for another 7 days and the process repeated. With this setup, approximately 300 - 450 milligrams of mixed hydrocarbon volatiles were recovered per week. The recovered extracts were combined and analysed on an Agilent J&W DB-624 60m (length) × 0.53 millimetre (inner diameter) × 3 micrometre (film thickness) (column 1) connected to a short inert column as described above (column 2). Carrier gas was helium at a constant flow rate of 3.8 millilitre / minute for column 1 and 1.8 millilitre / minute for column 2. Temperature programming was 110 °C for 11 minutes, then 20 °C/minute to 250 °C (7 minutes). The GC was interfaced with an Agilent 7000 GC/MS as described above. Automated sampling was performed by a Gerstel MultiPurpose Sampler using the proprietary Maestro software. An aliquot of 0.2 microlitre recovered volatiles was injected into the split/splittless injection port using a 5:1 split ratio.

The GC-MS analysis (0 to 65 minutes) identified 19 volatile metabolites produced by *Daldinia* sp. (U254) when grown for 14 days on OA at room temperature (Table 4). The metabolites produced by *Daldinia* sp. (U254) were representative of a number of structural classes, including alcohols (e.g. 3-pentanol), esters (e.g. ethyl acetate), terpenes and C7 aliphatic/aromatic unsaturated hydrocarbons and their associated derivatives (C7, e.g. 1-methyl-1,4-cyclohexadiene). The C7 compounds represent the major structural class within the volatolome, composing approximately one third of the compounds produced.

**Table 4 - GC-MS headspace analysis of the volatile compounds recovered from Daldinia sp. (U254) when grown on OA 14 days at room temperature.**

| **compound** | **match** | **RT** | **BP** | **significant minor ions (% of BP)** | **Relative intensity** |
|---|---|---|---|---|---|
| 2-Pentene (E) | 2 | 5.23 | 55.1 | 70.1 (75.44), 42.1 (31.77), 41.1 (16.73) | + |
| 2-Pentene (Z) | 2 | 5.3 | 55.1 | 70.1 (77.31), 42.1 (32.69), 41.1 (17.32) | + |
| Ethyl Acetate | 2 | 6.52 | 43.1 | 61.0 (30.59), 70.1 (21.67), 45.1 (16.08) | + |
| 3-Pentanol | 2 | 8.53 | 59.1 | 41.1 (20.09), 58.1 (14.1), | +++ |
| Cyclohexene, 1-methyl- | 2 | 9.26 | 81.0 | 96.0 (37.96), 67.0 (29.77), 54.1 (22.78) | ++ |
| Cyclohexene, 3-methyl- | 2 | 10.21 | 81.0 | 96.0 (48.13), 67.1 (46.95), 68.1 (33.88) | ++ |
| Toluene | 3 | 10.58 | 91.0 | 92.0 (71.82), 65.0 (9.05) | +++ |
| 1 -Methyl-1,4-cyclohexadiene | 3 | 11.59 | 79.0 | 94.0 (67.69), 77.0 (65.84), 91.0 (40.22) | +++ |
| Tricyclene | 2 | 15.21 | 93.0 | 91.0 (27.29), 92.0 (20.52), 79.0 (17.71) | + |
| α-Pinene | 2 | 15.34 | 93.0 | 92.0 (37.49), 91.0 (37.43), 77.0 (24.62) | +++ |
| 1-Cylohexane, 1-isopropyl-, 3-methyl- | 2 | 16.27 | 97.1 | 55.1 (84.53), 96.0 (67.06), 81.0 (38.4) | +++ |
| p-Menth-3-ene | 2 | 16.38 | 95.1 | 81.0 (77.71), 67.0 (39.15), 138 (33.05) | ++ |
| Cyclohexene, 1-methyl-4-(1-methylethyl)- | 1 | 16.49 | 95.0 | 81.0 (62.78), 67.0 (49.94), 68.1 (30.49) | ++ |
| 2-Carene | 1 | 17.16 | 121.0 | 93.0 (92.67), 136.0 (57.41), 91.0 (48.95) | +++ |
| p-Menth-1-ene | 1 | 17.38 | 95.0 | 81.0 (71.64), 67.1 (59.54), 68.1 (38.41) | +++ |
| Cymene | 2 | 17.48 | 119.0 | 134.0 (30.17), 91.0 (20.45), 117.0 (14.29) | +++ |

| | | | | | |
|---|---|---|---|---|---|
| 1 - Fragmentation pattern matches spectra of compound in NIST library (>75%) 2 - Fragmentation pattern matches spectra of compound in NIST library (>90%) 3 - Fragmentation pattern matches spectra and retention time of authentic standard + - significant peak; ++ - major peak; +++ - dominant peak | | | | | |

### Example 7 - Insecticidal activity of compounds from the volatolome of Daldinia sp.

A total of 20 chemical standards were evaluated for their insecticidal activity against the stored grain pest, *Tribolium castaneum.* These chemical standards represented compounds in the volatolome of *Daldinia* sp. or were structural analogues of these compounds (Table 5). The bioassays were conducted in 90 millimetre split Petri plates (as per example 5). The insect pest was inoculated on to one half of the Petri plate by placing 4 insects onto feed (wheat flour and yeast). A chemical standard was then aliquoted (5 microlitre) on to the other half of the Petri plate on filter paper. Plates were immediately sealed with Parafilm®, covered in aluminium foil (i.e. in the dark) and maintained at room temperature. The mortality of insects was monitored daily by assessing insect movement as an indicator of mortality. The mortality was calculated by comparing the number of dead insects to the total number in the plate, and expressed as percentage mortality (Tables 6). Data were analysed using ANOVA as performed in GenStat, version 14. The experiment was fully randomised with 5 replicates for each endophyte.

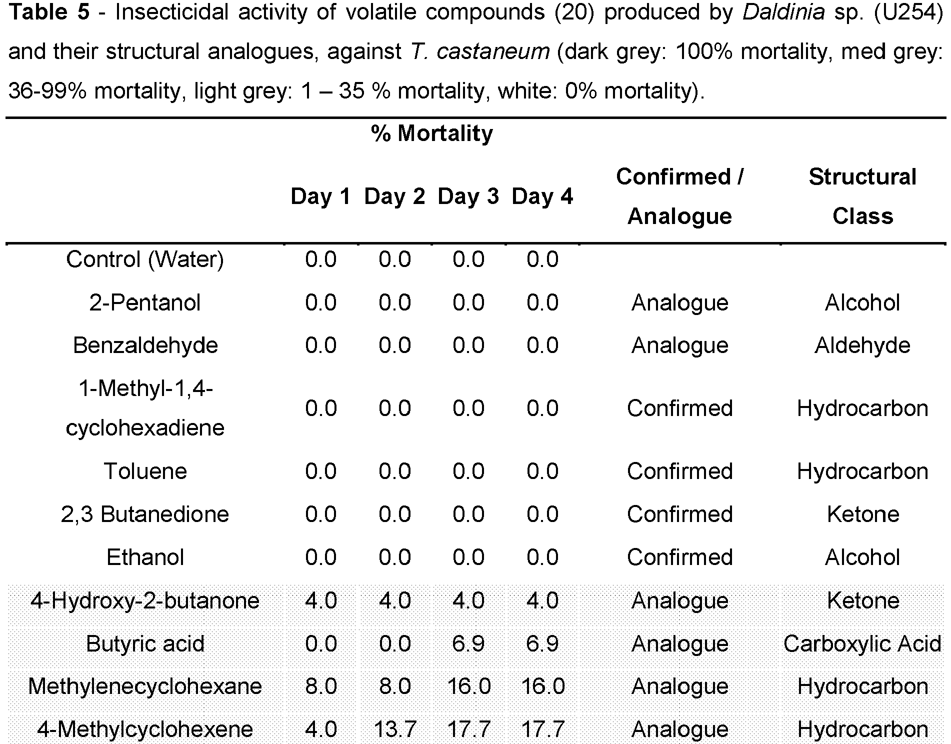

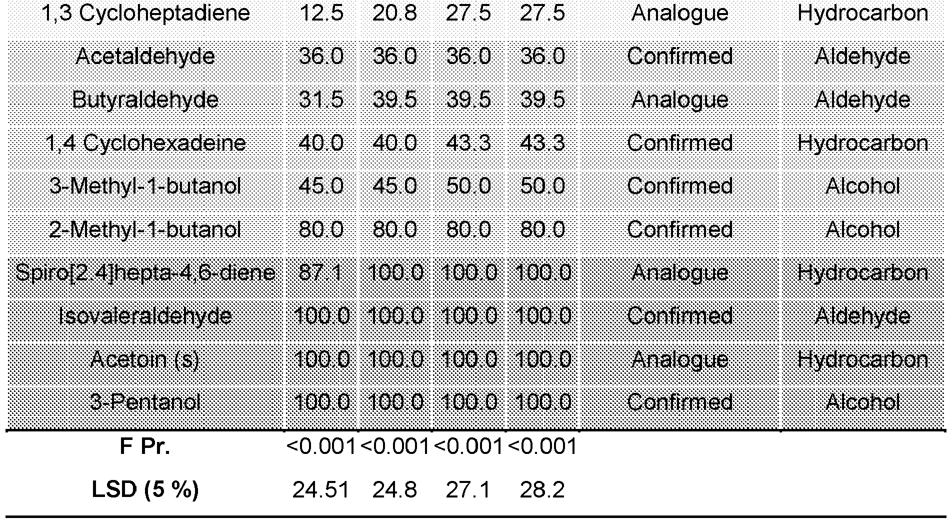

**Table 6 - Classification of the level of insecticidal activity of volatile compounds produced by Daldinia sp. and their structural analogues, against T. castaneum.**

| | **Mortality** | **Duration** | **No. of Compounds** |
|---|---|---|---|
| No activity | 0 % | | 6 |
| Low activity | 0 - 35 % | 24 - 96 hrs | 5 |
| Medium activity | 36 - 99% | 24 - 72 hrs | 5 |
| High activity | 100% | 24 - 48 hrs | 4 |

### Example 8 - Insecticidal dose response of key compounds from the volatolome of Daldinia sp. (U254)

An insecticidal dose response assay was established to evaluate four key compounds (3-pentanol, isovaleraldehyde acetoin and acetaldehyde) against the stored grain pest, *T. castaneum.* The bioassays were conducted in 1 litre Schott bottles. The insect pest was inoculated into the bioassay by placing 9 - 12 insects onto feed (wheat flour and yeast). The biocidal compounds were then aliquoted into the bioassay at volumes ranging from 20 - 200 microlitres, ensuring no direct contact with the insect. Bottles were immediately sealed with Parafilm® and maintained at room temperature. The mortality of insects was monitored daily for three days exposure, by assessing insect movement as an indicator of mortality. The mortality was calculated by comparing the number of dead insects to the total number in the bioassay, and expressed as percentage mortality. Data were analysed using ANOVA as performed in GenStat, version 14. The experiment was fully randomised with 4 replicates for each endophyte.

3-Pentanol exhibited the highest biocidal activity against *T. castaneum* of all compounds tested, with any volume ranging from 20 - 200 microlitres showing 100 % mortality after one day. Acetoin and Isovaleraldehyde exhibited biocidal activity against *T. castaneum* with volumes ranging from 20 - 200 microlitres, with volumes ranging from 50 - 200 microlitres showing 100 % mortality after a 1 day exposure. Acetaldehyde exhibited biocidal activity against *T. castaneum* with volumes ranging from 20 - 200 microlitres, and volumes ranging from 100 - 200 microlitres showing 98.2 - 100.0 % mortality after a 1 day exposure (Table 7).

**Table 7 - Dose response (20 - 200 microlitres) of four key compounds on T. castaneum after a 1 day exposure.**

| **Volume (µL)** | **Acetoin** | **Isovaleraldehyde** | **3-Pentanol** | **Acetaldehyde** |
|---|---|---|---|---|
| **0** | 0% | 0% | 0% | 0% |
| **20** | 3% | 3% | **100%** | 2.3% |
| **50** | **100%** | **100%** | 100% | 28.3% |
| **100** | 100% | 100% | 100% | 98.2% |
| **150** | 100% | 100% | 100% | **100%** |
| **200** | 100% | 100% | 100% | 100% |
| **F Pr.** | <0.001 | <0.001 | * | <0.001 |
| **LSD (5%)** | 3.42% | 3.10% | * | 12.55 |

### Example 9 - Bactericidal effect of 3-pentanol, acetoin and isovaleraldehyde, alone and in synergy with other key compounds from the volatolome of Daldinia sp. (U254)

A bactericidal bioassay was established to evaluate the effect of the most insecticidal candidate compounds against the plant pathogenic bacterium *Pseudomonas syringae,* when applied alone or in synergy with other compounds from the volatolome of *Daldinia* sp. The pathogen was inoculated on to one third of the Petri plate by streaking bacterial cells from an actively growing culture (overnight) on to nutrient agar (NA). A candidate compound was then aliquoted (10 microlitres - except Acetaldehyde: 20 microlitres due to high volatility) onto another third of the Petri plate on filter paper. In the synergy treatments, a second compound (acetaldehyde, 2-methyl-1-butanol, 1,4 cyclohexadiene, 1,3 cycloheptadiene, 4-methylcyclohexene) was added to the final third of the Petri plate on filter paper. An untreated control was included and consisted of no compounds. Plates were immediately sealed with Parafilm® and maintained at room temperature. After 3 days the growth of the pathogen was visually assessed and scored based on the following scale: 2 - equivalent growth to the control; 1 - less growth than the control; 0 - no growth. Compound combinations that exhibited 100 % inhibition were assessed for bacteristatic or bactericidal activity by subculturing from the original streak on to a fresh NA plate. Data were analysed using ANOVA as performed in GenStat, version 14. The experiment was fully randomised with 4 replicates for each compound combination.

Acetaldehyde and isovaleraldehyde exhibited high bactericidal activity against *P. syringae* when used alone or in combination. Acetoin and 3-pentanol did not exhibit any activity against *P. syringae* when used alone, but exhibited high bactericidal activity when combined with acetaldehyde. Isovaleraldehyde, when combined with acetaldehyde, 2-methyl-1-butanol, 1,4 cyclohexadiene, 1,3 cycloheptadiene or 4-methylcyclohexene completely inhibited the growth of *P. syringae* (Table 8). It is thought that the enhanced bioactivity of isovaleraldehyde and acetaldehyde when applied with other bioactive compounds could be attributed to different modes of action of the compounds, as they have diverse structures and are from varying chemical classes (alcohols, aldehydes, hydrocarbons).

**Table 8 - Bactericidal and synergistic effect of key Daldinia sp. (U254) compounds on biocidal activity against P. syringae.**

| | | **Isovaleraldehyde** | | **Acetoin** | | **3-Pentanol** | |
|---|---|---|---|---|---|---|---|
| | **Volume (µL)** | **Growth Scale** | **Activity** | **Growth Scale** | **Activity** | **Growth Scale** | **Activity** |
| **Control** | | 2 | | 2 | | 2 | |
| **Acetoin/Isoval/3-Pentanol** | **10** | 0.25 | - | 2 | - | 2 | - |
| **Acetaldehyde** | **10+20** | 0 | Bactericidal | 0.5 | Bactericidal | 0 | Bactericidal |
| **1,4-Cyclohexadeine** | **10+10** | 0 | Bactericidal | 2 | - | NA | - |
| **2-Methyl-1-butanol** | **10+10** | 0 | Bactericidal | 1 | - | 1 | - |
| **1,3-Cycloheptadeine** | **10+10** | 0 | Bactericidal | 2 | - | NA | - |
| **4-Methylcyclohexene** | **10+10** | 0 | Bactericidal | 2 | - | NA | - |
| **F Pr.** | | <0.001 | | <0.001 | | * | |
| **LSD (5%)** | | 0.32 | | 0.41 | | * | |

### Example 10 - Fungicidal activity of 3-pentanol, acetoin and isovaleraldehyde, alone and in synergy with key compounds from the volatolome of Daldinia sp. (U254)

A fungicidal bioassay was established to evaluate the effect of key compounds from the volatolome of *Daldinia* sp. against the plant pathogenic fungus, *Fusarium verticillioides,* when applied alone and in synergy with other compounds from the volatolome of *Daldinia* sp. The pathogen was inoculated on to one third of the Petri plate by placing an agar plug of actively growing hyphae onto PDA. Candidate compounds were then aliquoted (10 microlitres) on to another third of the Petri plate on filter paper. In the synergy treatments a second compound (acetaldehyde, 1,4-cyclohexadiene, 2-methyl-1-butanol, 1,3 cycloheptadiene, 4-methylcyclohexene) was added to the final third of the Petri plate on filter paper. Plates were immediately sealed with Parafilm® and maintained at room temperature. After 4 days the growth of the pathogen was determined by measuring the radius of the colony (on two alternate planes). Measurements were compared to the control and expressed as percentage inhibition relative to the control plate (no growth = 100 %). Compound combinations that exhibited 100 % inhibition were assessed for fungistatic or fungicidal activity by placing the original plug onto a fresh PDA plate. Data were analysed using ANOVA as performed in GenStat, version 14. The experiment was fully randomised with 4 replicates for each compound combination.

Isovaleraldehyde and acetaldehyde both exhibited high fungicidal activity against *F*. *verticillioides* in isolation and combination with any of the compounds tested, completely (100 %) inhibiting the growth of the pathogen (Table 9). In isolation, 3-pentanol exhibited moderate fungicidal activity against *F. verticillioides,* while Acetoin exhibited no activity. Both compounds showed 100 % inhibition when combined with Acetaldehyde, and varying rates of inhibition (7.4 % to 74.9 %) when combined with other compounds.

**Table 9 - Fungicidal and synergistic effect of key Daldinia sp. (U254) compounds on biocidal activity against F. verticillioides.**

| | | **Isovaleraldehyde** | | **Acetoin** | | **3-Pentanol** | |
|---|---|---|---|---|---|---|---|
| | **Volume (uL)*** | **%Inhibition (v control)** | **Activity** | **%Inhibition (v control)** | **Activity** | **%Inhibition (v control)** | **Activity** |
| **Acetoin/Isoval/3-Pentanol** | **10** | 100.0% | Fungicidal | 0 % | - | 47.3% | - |
| **Acetaldehyde** | **10+20** | 100.0% | Fungicidal | 100.0% | Fungicidal | 100.0% | Fungicidal |
| **1,4-Cyclohexadeine** | **10+10** | 100.0% | Fungicidal | 35.5% | - | NA | - |
| **2-Methyl-1-butanol** | **10+10** | 100.0% | Fungicidal | 52.2% | - | 74.9% | - |
| **1,3-Cycloheptadeine** | **10+10** | 100.0% | Fungicidal | 61.0% | - | NA | - |
| **4-Methylcyclohexene** | **10+10** | 100.0% | Fungicidal | 7.4% | - | NA | - |
| **F Pr.** | | * | | <0.001 | | * | |
| **LSD (5%)** | | * | | 21.41% | | * | |

It is to be understood that various alterations, modifications and/or additions may be made without departing from the spirit of the present invention as outlined herein.

As used herein, except where the context requires otherwise, the term "comprise" and variations of the term, such as "comprising", "comprises" and "comprised", are not intended to be in any way limiting or to exclude further additives, components, integers or steps.

Reference to any prior art in the specification is not, and should not be taken as, an acknowledgment or any form of suggestion that this prior art forms part of the common general knowledge in Australia or any other jurisdiction or that this prior art could reasonably be expected to be ascertained, understood and/or regarded as relevant by a person skilled in the art.

### References

1. Stadler, M., Læssøe, T., Fournier, J., Decock, C., Schmieschek, B., Tichy, H. V., & Peršoh, D. (2014). A polyphasic taxonomy of Daldinia (Xylariaceae). Studies in mycology, 77, 1-143.

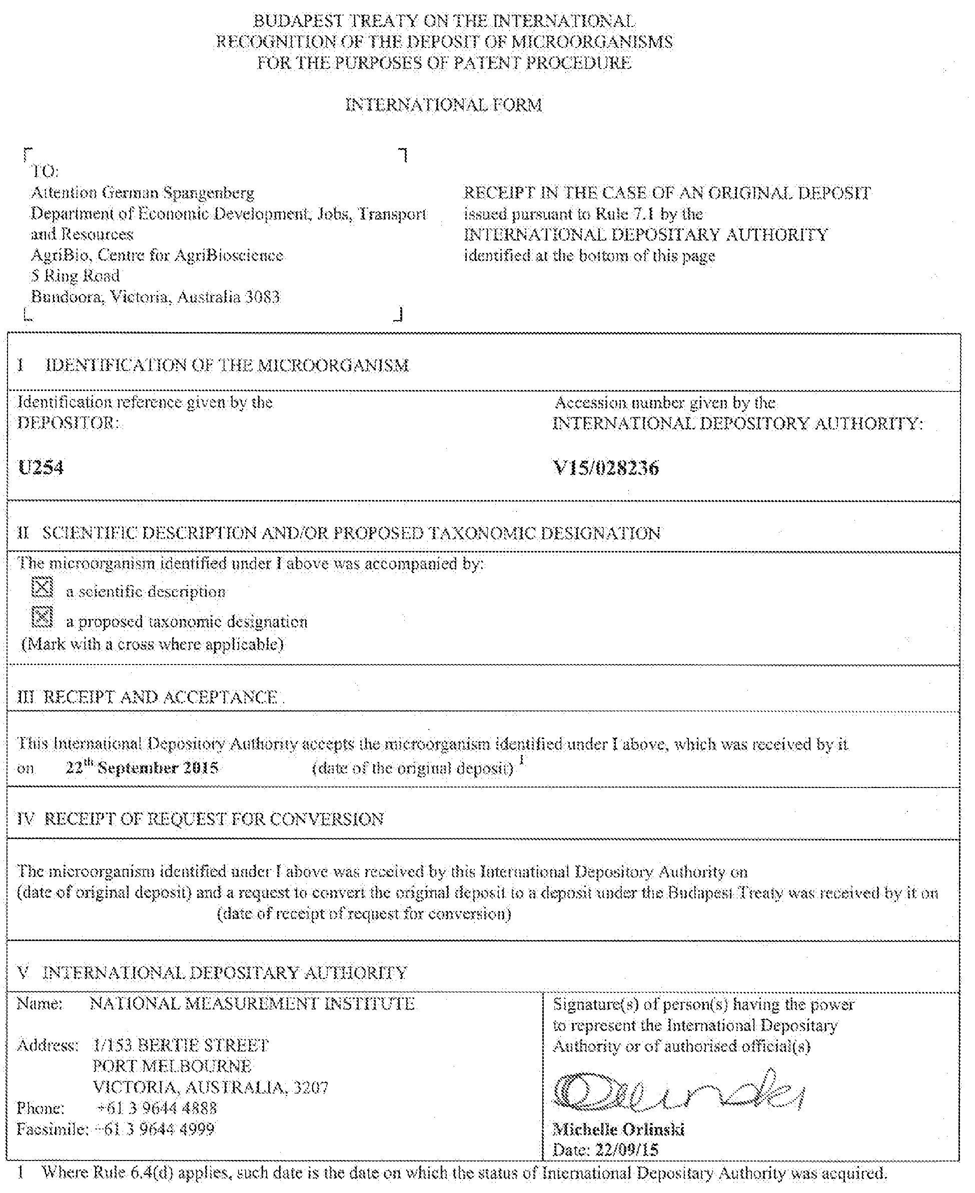

## Claims

1. A fungus of *Daldinia* sp. (U254) in axenic culture, as deposited at the National Measurement Institute under accession number V15/028236.

2. A method for producing an organic compound, said method including culturing a *Daldinia* spp. fungus according to claim 1, in a culture medium under conditions suitable to produce said organic compound and recovering an organic compound produced by the fungus from fungal cells, from the culture medium or from air space associated with the culture medium or fungus, and wherein said organic compound is a volatile organic compound.

3. A method according to claim 2, wherein the culture medium is selected from one or more of oatmeal agar (OA), half strength potato dextrose agar (HPDA), potato dextrose agar (PDA), endophyte agar (ENDO), Murashige and Skoog with 20% sucrose (MS-SUC), half V8 juice/half potato dextrose agar (V8PDA), water agar (WA) and yeast malt extract agar (YME).

4. A method according to claim 2 or 3, wherein said organic compound is selected from one or more of:
(a) the group consisting of: acetaldehyde, pentane, ethanol, 3-pentanol, acetone, 2,3-butanedione, isobutanol, ethyl acetate, isovaleraldehyde, 5,5-dimethyl-1,3-cyclopentadiene, 5,5-dimethyl-1,3-cyclopentadiene, bicyclo[4.1.0]hept-2-ene, 1-methyl-1,4-cyclohexadiene, (Z)-3-methyl-1,3,5-hexatriene, toluene, 4-methylphenol, 3-methyl-1-butanol, 2-methyl-1-butanol, (1Z)-3-methyl-1,3,5-hexatriene, (2Z)-3-methyl-1,3,5-hexatriene, p-xylene, styrene, dimethylcyclopentadiene, 1,4-cyclohexadiene, 4-heptyn-2-ol, 4-ethyl-1-octyn-3-ol, 2,2,4,6,6-pentamethyl-heptane, phenylethyl alcohol, guaiene, [1S-(1α,4α,7α)]-1,2,3,4,5,6,7,8-octahydro-1,4,9,9-tetramethyl-4,7-methanoazulene, (E)-2-pentene, (Z)-2-pentene, 1-methyl-cyclohexene, 3-methyl-cyclohexene, tricyclene, α-pinene,1-isopropyl-3-methylcyclohexane, p-menth-3-ene, 1-methyl-4-(1-methylethyl)-cyclohexene, 2-carene, p-menth-1-ene, cymene, terpenes and C7 aliphatic/aromatic unsaturated hydrocarbons; and
(b) a compound characterisable by about a base peak selected from the group consisting of a m/z of: 43.2, 59.1, 67.0, 68.1, 71.1, 79.0, 79.1, 81.0, 82.0, 91.0, 95.0, 97.0, 98.0, 108.0, 109.9, 115.0, 124.0, 132.9 and 192.8, or a base peak which is ± 0.1 of any of the foregoing, when analysed by mass spectrometry.

## Patentansprüche

1. Ein Pilz von *Daldinia* sp. (U254) in axenischer Kultur, wie beim National Measurement Institute unter der Zugangsnummer V15/028236 hinterlegt.

2. Ein Verfahren zur Herstellung einer organischen Verbindung, wobei jenes Verfahren das Kultivieren eines *Daldinia* spp. Pilzes nach Anspruch 1 in einem Kulturmedium unter Bedingungen, die zur Herstellung jener organischen Verbindung geeignet sind, und das Gewinnen einer vom Pilz aus Pilzzellen erzeugten organischen Verbindung aus dem Kulturmedium oder aus dem mit dem Kulturmedium oder Pilz verbundenen Luftraum einschließt, und wobei jene organische Verbindung eine flüchtige organische Verbindung ist.

3. Ein Verfahren nach Anspruch 2, wobei das Kulturmedium ausgewählt ist aus einem oder mehreren von Haferflockenagar (OA), Kartoffel-Dextrose-Agar halber Stärke (HPDA), Kartoffel-Dextrose-Agar (PDA), Endophyten-Agar (ENDO), Murashige und Skoog mit 20% Saccharose (MS-SUC), halb V8-Saft/halb Kartoffel-Dextrose-Agar (V8PDA), Wasser-Agar (WA) und Hefe-Malz-Extrakt-Agar (YME).

4. Ein Verfahren nach Anspruch 2 oder 3, wobei jene organische Verbindung ausgewählt ist aus einem oder mehreren von:
(a) die Gruppe bestehend aus: Acetaldehyd, Pentan, Ethanol, 3-Pentanol, Aceton, 2,3-Butandion, Isobutanol, Ethylacetat, Isovaleraldehyd, 5,5-Dimethyl-1,3-cyclopentadien, 5,5-Dimethyl-1,3-Cyclopentadien, Bicyclo[4.1.0]hept-2-en, 1-Methyl-1,4-cyclohexadien, (Z)-3-Methyl-1,3,5-hexatrien, Toluol, 4-Methylphenol, 3-Methyl-1-butanol, 2-Methyl-1-butanol, (1Z)-3-Methyl-1,3,5-hexatrien, (2Z)-3-Methyl-1,3,5-hexatrien, p-Xylol, Styrol, Dimethylcyclopentadien, 1,4-Cyclohexadien, 4-Heptyn-2-ol, 4-Ethyl-1-octyn-3-ol, 2,2,4,6,6-Pentamethylheptan, Phenylethylalkohol, Guaien, [1S-(1α,4α,7α)]-1,2,3,4,5,6,7,8-Octahydro-1,4,9,9-tetramethyl-4,7-methanoazulen, (E)-2-Penten, (Z)-2-Penten, 1-Methylcyclohexen, 3-Methylcyclohexen, Tricyclen, α-Pinen, 1-Isopropyl-3-methylcyclohexan, p-Menth-3-en, 1-Methyl-4-(1-methylethyl)-cyclohexen, 2-Caren, p-Menth-1-en, Cymol, Terpene und aliphatische/aromatische ungesättigte C7-Kohlenwasserstoffe; und
(b) eine Verbindung, die durch etwa einen Basenpeak charakterisiert ist, ausgewählt aus der Gruppe bestehend aus einem m/z von: 43,2, 59,1, 67,0, 68,1, 71,1, 79,0, 79,1, 81,0, 82,0, 91,0, 95,0, 97,0, 98,0, 108,0, 109,9, 115,0, 124,0, 132,9 und 192,8 oder einen Basispeak, der ± 0,1 eines der vorstehenden Werte beträgt, wenn er durch Massenspektrometrie analysiert wird.

## Revendications

1. Champignon de *Daldinia* sp. (U254) en culture axénique, tel que déposé auprès du National Measurement Institute sous le numéro d'accès V15/028236.

2. Procédé de production d'un composé organique, ledit procédé comportant la culture d'un champignon *Daldinia* spp. selon la revendication 1, dans un milieu de culture dans des conditions adaptées pour produire ledit composé organique et récupérer un composé organique produit par le champignon à partir de cellules fongiques, à partir du milieu de culture ou à partir d'un espace d'air associé au milieu de culture ou au champignon, et dans lequel ledit composé organique est un composé organique volatil.

3. Procédé selon la revendication 2, dans lequel le milieu de culture est sélectionné parmi un ou plusieurs d'une gélose à l'avoine (OA), d'une gélose dextrosée à la pomme de terre diluée à 50 % (HPDA), d'une gélose dextrosée à la pomme de terre (PDA), d'une gélose d'endophyte (ENDO), du milieu de Murashige et Skoog avec 20 % de saccharose (MS-SUC), d'une gélose dextrosée moitié au jus V8/moitié à la pomme de terre (V8PDA), d'une gélose à l'eau (WA) et d'une gélose à l'extrait de malt pour levures (YME).

4. Procédé selon la revendication 2 ou 3, dans lequel ledit composé organique est sélectionné parmi un ou plusieurs de :
(a) le groupe consistant en : l'acétaldéhyde, le pentane, l'éthanol, le 3-pentanol, l'acétone, la 2,3-butanedione, l'isobutanol, l'acétate d'éthyle, l'isovaléraldéhyde, le 5,5-diméthyl-1,3-cyclopentadiène, le 5,5-diméthyl-1,3-cyclopentadiène, le bicyclo[4.1.0]hept-2-ène, le 1-méthyl-1,4-cyclohexadiène, le (Z)-3-méthyl-1,3,5-hexatriène, le toluène, le 4-méthylphénol, le 3-méthyl-1-butanol, le 2-méthyl-1-butanol, le (1Z)-3-méthyl-1,3,5-hexatriène, le (2Z)-3-méthyl-1,3,5-hexatriène, le p-xylène, le styrène, le diméthylcyclopentadiène, le 1,4-cyclohexadiène, le 4-heptyn-2-ol, le 4-éthyl-1-octyn-3-ol, le 2,2,4,6,6-pentaméthyl-heptane, l'alcool phényléthylique, le guaiène, le [1S-(1α,4α,7α)]-1,2,3,4,5,6,7,8-octahydro-1,4,9,9-tétraméthyl-4,7-méthanoazulène, le (E)-2-pentène, le (Z)-2-pentène, le 1-méthyl-cyclohexène, le 3-méthyl-cyclohexène, le tricyclène, l'α-pinène, le 1-isopropyl-3-méthylcyclohexane, le p-menth-3-ène, le 1-méthyl-4-(1-méthyléthyl)-cyclohexène, le 2-carène, le p-menth-1-ène, le cymène, les terpènes et les hydrocarbures insaturés aliphatiques/aromatiques en C7 ; et
(b) un composé caractérisable par environ un pic de base sélectionné dans le groupe consistant en un m/z de : 43,2, 59,1, 67,0, 68,1, 71,1, 79,0, 79,1, 81,0, 82,0, 91,0, 95,0, 97,0, 98,0, 108,0, 109,9, 115,0, 124,0, 132,9 et 192,8, ou un pic de base qui est ± 0,1 de l'un quelconque des précédents, lorsqu'il est analysé par spectrométrie de masse.
